# EUROPÄISCHE PATENTSCHRIFT

(11) **EP 1 022 565 B1**
(45) Veröffentlichungstag und Bekanntmachung des Hinweises auf die Patenterteilung: **30.05.2007**
(21) Anmeldenummer: 00100255.9
(22) Anmeldetag: 18.01.2000
(51) Int. Cl.: G01N 33/48, B01L 11/00, G01N 35/00, G01N 33/487, B23Q 5/40, F16H 25/12

(54) **Verfahren und Vorrichtung zum Entnehmen analytischer Verbrauchsmittel aus einem Vorratsbehältnis**
Method and device for withdrawing analytical consumables from a storage container
Dispositif et méthode pour retirer des éléments d'analyse à usage unique d'un récipient de stockage

(30) Priorität: 23.01.1999 DE 19902601
(43) Veröffentlichungstag der Anmeldung: 26.07.2000
(73) Patentinhaber: Roche Diagnostics GmbH, 68305 Mannheim (DE)
(72) Erfinder: Schabbach, Michael, 69493 Hirschberg (DE); Miltner, Karl, 67227 Frankenthal (DE); Klemm, Thomas, 68239 Mannheim (DE); Baumann, Rolf, 78052 Rietheim, Villingen-Schwenningen (DE); Dilger, Meinrad, 78112 St. Georgen (DE)
(74) Vertreter: Jany, Peter

(56) Entgegenhaltungen:
- EP-A- 0 357 935
- US-A- 5 332 549
- US-A- 5 510 266
- N.M.SCHMIDT ET AL.: "understanding automation systems" 1984 , TEXAS INSTRUMENTS , USA XP002238432 * Seite 60, Absatz 2 *

## Beschreibung

Die Erfindung betrifft eine Entnahme vorrichtung zum Entnehmen eines analytischen Verbrauchsmittels, insbesondere eines Testelements, aus einem Vorratsbehältnis, das ein oder mehrere Kammern aufweist, und ein entsprechendes Analysegerät. Die Kammern enthalten jeweils ein oder mehrere Verbrauchsmittel und weisen jeweils eine Entnahmeöffnung zum Entnehmen eines Verbrauchsmittels und eine der Entnahmeöffnung gegenüberliegende Einschuböffnung zum Einführen eines Stößels für den Transport des zu entnehmenden Verbrauchsmittels auf. Dabei sind die Entnahmeöffnung und die Einschuböffnung zur Lagerung des Verbrauchsmittels mit einer Folie, die auch als Siegelfolie bezeichnet wird, verschlossen. Zur Entnahme eines Verbrauchsmittels wird ein Stößel mittels einer Antriebseinheit verschoben und das Verbrauchsmittel durch den Stößel aus der Kammer in dem Vorratsbehältnis herausbefördert.

Für die chemische und biochemische Analyse von festen und flüssigen Probenmaterialien haben sich in darauf spezialisierten Labors und insbesondere auch für den Einsatz außerhalb fester Labors trägergebundene Schnelltests etabliert. Solche trägergebundenen Schnelltests basieren auf einer eigens entwickelten Trockenchemie und sind trotz der oftmals komplexen Reaktion unter Beteiligung empfindlicher Reagenzien selbst von Laien einfach und unkomplziert durchzuführen.

Ein bekanntes Beispiel für trägergebundene Schnelltests sind Testelemente für die Bestimmung des Blutglucosegehaltes bei Diabetikern. Diagnostische Testelemente, die streifenförmig ausgebildet sind, werden auch als Teststreifen bezeichnet. Bekannte Ausführungsformen sind z.B. Ein- oder Mehrfelderteststreifen für die Urinanalytik und diverse Indikatorpapiere. Da neben Testelementen in Streifenform auch andere Formen trägergebundener Tests exisitieren, spricht man allgemeiner von analytischen Testelementen.

Analytische Testelemente in dem erfindungsgemäßen Zusammenhang sind visuell oder apparativ auswertbar. Apparativ auswertbare Testelemente sind beispielsweise optisch, insbesondere photometrisch auswertbare Testelemente oder elektrochemische Sensoren und dergleichen mehr. Derartige analytische Testelemente sind, wie andere analytische Verbrauchsmittel, in einem Vorratsbehältnis verpackt, um sie vor schädlichen Umwelteinflüssen wie z.B. Licht, Feuchtigkeit oder mechanischer Einwirkung zu schützen oder unter sterilen Bedingungen aufzubewahren. Zu den analytischen Verbrauchsmitteln zählen neben den Testelementen beispielsweise auch Lanzetten oder Probennahmeelemente.

Da derartige analytische Verbrauchsmittel im Stand der Technik umfassend beschrieben und dem Fachmann in einer Vielzahl von Ausführungsformen geläufig sind, erübrigt sich hier eine detaillierte Beschreibung. Statt dessen sei beispielsweise auf folgende Dokumente verwiesen: DE-A 19753847.9, EP-A 0138152, EP-A 0821233, EP-A 0821234, EP-A 0630609, EP-A 0565970 und WO 97/02487.

Analytische Verbrauchsmittel werden in einem Vorratsbehältnis aus einem starren Material gelagert, um sie vor der Einwirkung von Lichtstrahlen, dem Zutritt von Luftfeuchtigkeit, Schmutz, Keimen und Staub sowie vor mechanischer Beeinträchtigung zu schützen. Wenn das Vorratsbehältnis mehrere Verbrauchsmittel enthält, sind diese zumeist in einzelnen Kammern untergebracht, wobei die Kammern jeweils ein oder mehrere Verbrauchsmittel enthalten können. Es können in einem Vorratsbehältnis auch verschiedene Arten analytischer Verbrauchsmittel, z.B. Testelemente und Lanzetten, in jeweils eigenen Kammern enthalten sein.

Zur Entnahme eines Verbrauchsmittels wird eine der Kammern geöffnet, wobei die Entnahmeöffnung und die Einschuböffnung verschließende Folien geöffnet werden. Auf diese Weise ist es möglich, bedarfsweise Verbrauchsmittel aus Kammern zu entnehmen, ohne die anderen Kammern zu öffnen, so daß die in den ungeöffneten Kammern enthaltenen Verbrauchsmittel weiter sicher gelagert werden können.

Die Vorratsbehältnisse und Kammern können in verschiedener Weise ausgestaltet sein und enthalten in vielen Fällen einen Trockenmittelvorrat, um den Schutz vor Feuchtigkeit zu erhöhen. Die Vorratsbehältnisse können mit einem Datenträger, beispielsweise einem Etikett in lesbarer Schrift, einem Strichcode-Etikett oder einem Magnetstreifen versehen sein, auf dem chargenspezifische Daten und ggf. weitere Informationen zu dem analytischen Verbrauchsmittel gespeichert und abrufbar sind.

Die analytischen Verbrauchsmittel können manuell oder vorzugsweise durch eine mechanische Vorrichtung aus dem Vorratsbehältnis entnommen werden, wobei die in dem Vorratsbehältnis in ungeöffneten Kammern verbleibenden Verbrauchsmittel durch die Einzelversiegelung mittels der Folie weiterhin geschützt sind. Die Entnahme der Verbrauchsmittel erfolgt durch Herausschieben aus der Kammer mit Hilfe eines Stößels.

Vorratsbehältnisse für analytische Verbrauchsmittel und die entsprechenden Vorrichtungen zum Entnehmen der Verbrauchsmittel sind im Stand der Technik umfangreich beschrieben und dem Fachmann in einer Vielzahl von Ausführungsformen geläufig. In diesem Zusammenhang wird beispielsweise auf folgende Dokumente verwiesen: EP-A 0622119, EP-A 0732590, EP-A 0738666, US 5,489,414, US 5,510,266, US 5,720,924 und insbesondere US 5,632,410 sowie DE-A 19854316.

Die Vorratsbehältnisse, die auch als Magazine bezeichnet werden, sind zumeist für den Einsatz in Meßgeräten, insbesondere in kompakten Meßgeräten konzipiert. Für die Aufnahme eines Vorratsbehältnisses in ein Meßgerät, in dem mit Hilfe eines Stößels ein Verbrauchsmittel aus dem Vorratsbehältnis entnommen wird, können entsprechende Mittel, insbesondere zum genauen Positionieren des Vorratsbehältnisses relativ zu funktionalen Bestandteilen eines Analysegerätes und hierbei insbesondere zum Stößel für die Verbrauchsmittelentnahme vorgesehen sein.

Die Entnahme eines Verbrauchsmittels ist in vielen Ausführungsformen automatisiert, beispielsweise um Fehlbedienungen auszuschließen oder um die Bedienungsfreundlichkeit zu erhöhen. In diesen Fällen wird der die Entnahme eines Verbrauchsmittels bewirkende Stößel mittels einer Antriebseinheit, die einen elektrischen Antriebsmotor und eventuell ein Getriebe umfaßt, bewegt. Beispiele konventioneller manueller, motorischer und automatisierter Vorrichtungen zum Entnehmen analytischer Verbrauchsmittel aus Vorratsbehältnissen sind in den oben genannten Dokumenten beschrieben.

Charakteristisch für die Vorratsbehältnisse, auf die sich die Erfindung bezieht, ist, daß sie an zwei gegenüberliegenden Öffnungen jeweils mit einer Folie verschlossen sind, die bei der Entnahme des Verbrauchsmittels durchstoßen werden müssen. Zunächst dringt der Stößel durch die Folie über die Einschuböffnung in die Kammer des Vorratsbehältnisses und drückt dort das zu entnehmende Verbrauchsmittel weiter. Anschließend wird durch das in Vorschubrichtung vordere Ende des Verbrauchsmittels die Folie über der Entnahmeöffnung nach außen hin aufgerissen und das Verbrauchsmittel aus der Kammer herausgeschoben bzw. in eine Gebrauchsstellung gebracht. Dieser Transportvorgang bringt es mit sich, daß in Teilbereichen des Transportweges relativ hohe Kräfte benötigt werden (z.B. beim Durchstoßen der beiden Folien oder beim Plazieren eines Testelements in einer vorgegebenen Position einer Meßhalterung), wogegen auf dem übrigen Transportweg nur eine relativ geringe Vorschubkraft benötigt wird.

Die Auswahl für Material und Dicke der Folien, die zum Verschließen der Öffnungen der Kammern des Vorratsbehältnisses dienen, ist durch zwei Erfordernisse eingeschränkt. Einerseits müssen sie hinreichend fest sein, um einen ausreichenden Schutz zu bieten und keine mechanische Schwachstelle bei der Handhabung des Vorratsbehältnisses darzustellen. Andererseits darf die Folie nicht zu fest sein, damit sie durch den Stößel bzw. durch das analytische Verbrauchsmittel durch den von der Vorschubkraft des Stößels ausgehenden Druck durchtrennt werden kann.

Inbesondere in kleinen, kompakten Analysegeräten, die batteriebetrieben sind, bestehen zudem die widersprüchlichen Anforderungen, daß einerseits die Geschwindigkeit, mittels der die Entnahme eines Verbrauchsmittels, insbesondere eines Testelements durchgeführt wird, hoch sein soll und andererseits der damit verbundene Batterieverbrauch gering sein soll, um mit einem Batteriesatz möglichst viele Messungen durchführen zu können.

Nach dem Stand der Technik werden diese technischen Probleme hinsichtlich der aufzubringenden Kraft, der Schnelligkeit der Verbrauchsmittelentnahme und des Energiebedarfs dadurch gelöst, daß die Antriebseinheit eine resultierende konstante Vorschubkraft des Stößels erzeugt, die sich aus der Motorkraft des Antriebsmotors und einer eventuellen Getriebeübersetzung ergibt und die hoch genug ist, um die im Transportweg auftretenden Lastspitzen zu bewältigen. Ob dies durch die Wahl eines kräftigeren Antriebsmotors mit entsprechend höherem Batterieverbrauch oder durch die Wahl einer anderen Getriebeübersetzung geschieht, ist von der jeweiligen Auslegung abhängig, d.h. davon, ob eine rasche Meßfolge oder ein geringer Gesamtbatterieverbrauch wichtiger ist.

Der Antrieb wird nach dem Stand der Technik somit nach der maximal auftretenden Last ausgelegt und zwischen den beiden Extremen minimaler Energieverbrauch und maximale Transportzeit einerseits oder maximaler Energieverbrauch und minimale Transportzeit andererseits optimiert. In keinem Fall wird jedoch nach dem Stand der Technik eine wirklich optimale Lösung erzielt, da der Antrieb über weite Strecken des Vorschubweges des Verbrauchsmittels die Anforderungen hinsichtlich Schnelligkeit und Energieverbrauch nicht beide optimal erfüllen kann.

Aus dem Dokument US 5,332,549 ist ein Analysegerät bekannt, bei dem mittels einer Entnahmevorrichtung aus einem Trommelmagazin ein Verbrauchsmittel entnommen wird. Die Vorschubkraft bzw. der für den Entnahmevorgang benutzte Schrittmotor wird mittels eines Mikroprozessors gesteuert. Die Eigenschaften von Schrittmotoren sind beispielsweise in dem Dokument N.M. Schmidt and R.F. Farwell, Understanding automation systems, Texas Instruments 1984, Seite 60 beschrieben.

Ausgehend von diesem Stand der Technik liegt der Erfindung die Aufgabe zugrunde, eine eingangs genannte Vorrichtung zum Entnehmen eines analytischen Verbrauchsmittels, insbesondere eines Testelements, aus einem Vorratsbehältnis dahingehend zu verbessern, daß die Anforderungen hinsichtlich Schnelligkeit der Entnahme des Verbrauchsmittels sowie der Minimierung eines damit verbundenen Energieverbrauchs gleichzeitig verbessert werden, insbesondere bei sehr kompakter Bauweise einer entsprechenden Vorrichtung.

Zur Lösung dieser Aufgabe wird eine Vorrichtung mit den Merkmalen des Anspruchs 1 vorgeschlagen.

Im Rahmen der vorliegenden Erfindung wurde überraschenderweise festgestellt, daß die außerordentlich schwierigen Anforderungen bei Vorrichtungen zum Entnehmen eines analytischen Verbrauchsmittels aus Vorratsbehältnissen mit einem Stößel, dessen Vorschubkraft sich als Funktion des Vorschubweges ändert, gelöst werden können. Ferner hat sich gezeigt, daß eine solche weg- bzw. ortsabhängige mit einem Stößel, dessen Vorschubkraft sich als Funktion des Vorschubweges ändert, gelöst werden können. Ferner hat sich gezeigt, daß eine solche weg- bzw. ortsabhängige Vorschubkraft mit einfachen Mitteln gelöst werden kann, so daß die gesamte Entnahme gleichzeitg hinsichtlich beider Anforderungen betreffend Geschwindigkeit und Energieaufwand optimiert werden kann und nicht, wie es bisher für erforderlich gehalten wurde, nur jeweils hinsichtlich einer Anforderung bzw. im Wege einer Kompromißlösung.

Vergleichsversuche haben beispielsweise ergeben, daß ein konventioneller Antrieb, der auf Energieeinsparung optimiert ist, eine Transportzeit zur Entnahme eines Teststreifens aus einem trommelförmigen Magazin 20 sec benötigt und mit einem batteriebetriebenen Meßgerät mehr als 500 Teststreifen gemessen werden können. Wenn der Antrieb dagegen auf eine schnelle Geschwindigkeit der Entnahme optimiert wird, wobei die Transportzeit ca. 4 sec beträgt, können nur 50 Teststreifen pro Batteriesatz gemessen werden. Mit einer erfindungsgemäß ausgebildeten Vorrichtung dagegen liegt die Transportzeit zwischen 4 und 5 sec, wobei ebenfalls mehr als 500 Teststreifen je Batteriesatz gemessen werden können.

Mit der Erfindung werden somit Ziele erreicht, um die die Fachwelt sich schon lange bemüht hat. Ferner sind die Beschränkungen hinsichtlich des Materials und der Dicke der Folien bei einer erfindungsgemäßen Vorrichtung weniger bedeutsam, so daß kostengünstigere oder für den jeweiligen Einsatzzweck geeignetere Materialien in einer größeren Auswahl zur Verfügung stehen.

Um besonders gute Ergebnisse hinsichtlich Schnelligkeit der Entnahme eines Verbrauchsmittels, des damit verbundenen Energieverbrauchs sowie hinsichtlich der konstruktiven Erfordernisse und der damit verbundenen Kosten zu gewährleisten, werden bevorzugt die nachfolgenden Maßnahmen einzeln oder in Kombination miteinander eingesetzt.

Nach einem ersten bevorzugten Merkmal kann vorgesehen sein, daß die Vorschubkraft in Abhängigkeit von dem Vorschubweg steuerbar ist, den der Stößel zurückgelegt hat. Dabei wird bevorzugt der Vorschubweg relativ zu einem Teil mit einer bezüglich der Vorrichtung festen Postion bestimmt. Dies kann beispielsweise die Anfangsposition des Stößels, die Endposition des Stößels oder die Lage einer der Folien sein. Alternativ besteht auch die Möglichkeit, eine absolute Positionsmessung des Stößels durchzuführen.

Ferner läßt sich die Entnahme eines analytischen Verbrauchsmittels vorteilhafterweise dadurch verbessern, daß die Vorschubkraft bei mindestens einem der folgenden Betriebszustände erhöht ist: beim Durchstoßen der Folie über der Einschuböffnung durch den Stößel, beim Durchstoßen der Folie über der Entnahmeöffnung durch das Verbrauchsmittel, beim Positionieren des entnommenen Verbrauchsmittels in eine vorgegebene Gebrauchslage oder beim Ausstoßen eines verbrauchten Verbrauchsmittels aus einer vorgegebenen Gebrauchslage.

Eine Gebrauchslage in diesem Sinne ist jede bestimmte, definierte Position, die ein Verbrauchsmittel für seinen bestimmungsgemäßen Gebrauch einnehmen muß, beispielsweise der Ort einer Probenaufnahme oder eine Position, in der eine analytische Messung durchgeführt wird. In solchen Gebrauchslagen müssen die Verbrauchsmittel in aller Regel genau positioniert werden, wozu entsprechende Führungen oder Anschlagelemente vorgesehen sind, die eine Erhöhung der zum Transport des Gebrauchsmittel erforderlichen Vorschubkraft zur Folge haben. Allgemein kann gesagt werden, daß mit der erfindungsgemäßen Vorrichtung die Vorschubkraft in den Bereichen, in denen eine erhöhte Last auftritt, beispielsweise in den vorstehend genannten Positionen, erhöht werden kann, wogegen die Vorschubkraft in anderen Bereichen des Vorschubweges des Stößels niedriger ausgelegt werden kann. Dadurch wird insbesondere der Energieverbrauch minmiert, da nur in solchen Bereichen, in denen der Stößel eine erhöhte Last überwinden muß, eine erhöhte Vorschubkraft zur Verfügung gestellt wird.

Nach einem anderen bevorzugten Merkmal ist die Vorrichtung derart ausgebildet, daß die Vorschubgeschwindigkeit des Stößels in Bereichen mit erhöhter Vorschubkraft reduziert und in Bereichen mit reduzierter Vorschubkraft erhöht ist. Bei Berücksichtigung dieser Regel läßt sich die für die Entnahme des Verbrauchsmittels erforderliche Gesamtzeit des Transportes unter Berücksichtigung des damit verbundenen Energieverbrauchs besonders gut optimieren.

Eine gewünschte Vorschubkraft-Vorschubweg-Charakteristik läßt sich prinzipiell auf rein elektronischem Wege realisieren, wobei die Vorschubbewegung des Stößels mittels einer elektronischen Regelung der Antriebseinheit gesteuert wird. Hierfür können beispielsweise die Leistung oder die Drehzahl des Antriebsmotors oder ein regelbares Getriebe gesteuert werden. Andere Möglichkeiten einer elektronischen Regelung bestehen in der Verwendung von Schrittmotoren, elektronisch kommutierten Motoren, einer Stromregelung des Antriebsmotors oder einer Pulsweitenmodulation des Antriebsmotors oder ähnlichen Verfahren.

Elektronische Regelungen können den Vorteil haben, daß die Vorschubgeschwindigkeit unabhängig von der Betriebsspannung wird, weisen aber in aller Regel den Nachteil auf, daß ein höherer konstruktiver Aufwand erforderlich ist und der Antriebsmotor in den meisten Fällen nicht in einem optimalen Arbeitspunkt gehalten werden kann.

Nach einem bevorzugten Merkmal der Erfindung wird nämlich vorgeschlagen, daß die Antriebseinheit einen Antriebsmotor umfaßt, der mit einer im wesentlichen konstanten Antriebsleistung und/oder im wesentlichen konstanten Drehzahl betreibbar ist, um eine möglichst optimale Energieausnutzung dadurch zu erzielen, daß die Stromquelle, beispielsweise eine Batterie oder ein Akkumulator, gleichmäßig belastet und der Antriebsmotor bei einem Arbeitspunkt mit einem guten Wirkungsgrad betrieben wird.

Im Rahmen der Erfindung hat sich gezeigt, daß diese Ziele bevorzugt erreicht werden, wenn die Vorschubbewegung des Stößels mittels einer mechanischen Wegsteuerung, insbesondere einer mechanischen Kurvensteuerung gesteuert wird. Eine mechanische Wegsteuerung bzw. Kurvensteuerung kann zudem die weiteren Vorteile aufweisen, daß sie konstruktiv einfach und kostengünstig ist, geringe Reibungsverluste aufweist oder die Transportposition des Stößels entlang des Vorschubweges und die Vorschubkraft bzw. die Vorschubgeschwindigkeit in Abhängigkeit von der Position des Stößels durch eine Wirkverbindung zwischen Kurvensteuerung und Stößel realisiert werden können, ohne daß hierzu wie zumeist bei einer elektronischen Lösung ein gesonderter Weg- oder Positionsaufnehmer erforderlich ist. Durch eine Wirkverbindung zwischen Weg- bzw. Kurvensteuerung und Stößel kann eine Koppelung zwischen Position des Stößels und Vorschubkraft bzw. Vorschubgeschwindigkeit erzielt werden.

Eine mechanische Weg- bzw. Kurvensteuerung kann auf verschiedene Weise ausgebildet sein. Nach einem erfindungsgemäßen Merkmal umfaßt die Weg-bzw. Kurvensteuerung ein schraubenförmig gewundenes, mittels der Antriebseinheit um seine Längsachse in Drehbewegung versetzbares und den Vorschub des Stößels steuerndes Steuerelement , das in Verbindung mit einem Mitnehmerteil steht, das den Stößel beim Drehen des Steuerelements in Vorschubrichtung bewegt. Auf diese Weise wird die von der Antriebseinheit erzeugte Rotation des Steuerelementes in eine lineare Bewegung des Stößels umgesetzt.

Eine konstruktiv einfache Lösung kann dabei nach einem zusätzlichen vorteilhaften Merkmal dann realisiert werden, wenn sich die Längsachse des Steuerelements in Richtung der Vorschubbewegung des Stößels erstreckt. Der Stößel kann dabei parallel neben dem Steuerelement angeordnet sein oder nach einem bevorzugten Merkmal das Steuerelement axial durchdringen, wodurch eine besonders kompakte Bauweise erzielt wird.

Hinsichtlich der Steigung des Steuerelements der Weg- bzw. Kurvensteuerung kann vorgesehen sein, daß diese längs der schraubenförmigen Windung konstant ist. In diesem Fall ist zur Erzielung einer ortsabhängigen Vorschubkraft das Antriebsmoment der Antriebseinheit zu steuern und zur Erzielung einer ortsabhängigen Vorschubgeschwindigkeit die Drehzahl des Steuerelementes zu variieren.

Bevorzugt ist eine Ausführungsform, bei der die Steigung des Steuerelements längs seiner schraubenförmigen Windung entsprechend einer gewünschten Vorschubkraft- Positionsabhängigkeit des Stößels variabel ausgebildet ist. In diesem Fall kann nach einem zusätzlichen, besonders vorteilhaften Merkmal die Drehzahl des Antriebsmotors der Antriebseinheit bzw. die Drehzahl des Steuerelements beim Vorschub des Stößels im wesentlichen konstant sein.

Ein erfindungsgemäßes, schraubenförmig gewundenes Steuerelement kann auf vielfältige Weise realisiert werden. Eine erste vorteilhafte Ausbildung kann darin bestehen, daß das Steuerelement eine zylinderförmige Steuerwalze mit einer auf der Mantelfläche verlaufenden Nut und das Mitnehmerteil einen in die Nut eingreifenden Nutenstein umfaßt. Die Nut definiert eine Vorschubkurve mit der gewünschten Charakteristik. Derartige Konstruktionen sind in Form eines Spindeltriebes für Regalförderzeuge aus dem Dokument EP-A 0357935 bekannt. Sie sind jedoch für den erfindungsgemäßen Zweck, insbesondere für kompakte Analysegeräte, relativ groß.

Besonders bevorzugt ist daher die Realisierung eines schraubenförmig gewundenen Steuerelements in Form einer zylindrischen, schraubenförmig gewundenen Transportwendel, wobei das Mitnehmerteil als in die Windungen der Transportwendel eingreifender Mitnehmerstift ausgebildet ist. Beim Drehen der Transportwendel wird entsprechend der Steigung der Transportwendel und der Drehgeschwindigkeit der Mitnehmerstift in axialer Richtung der Transportwendel transportiert. Der Mitnehmerstift steht in Wirkverbindung mit dem Stößel, so daß der Stößel je nach Drehrichtung der Transportwendel vor- oder zurückbewegt wird, wobei die Vorschubkraft und die Vorschubgeschwindigkeit durch die Transportwendel bestimmt werden. Auf diese Weise kann eine konstruktiv besonders unaufwendige erfindungsgemäße Vorrichtung realisiert werden.

Ein erfindungsgemäßes Analysegerät zur Analyse einer medizinischen Probe mittels eines medizinischen Verbrauchsmittels, insbesondere zur Durchführung einer Analyse mittels eines Testelements, ist dadurch gekennzeichnet, daß es eine erfindungsgemäße Vorrichtung zum Entnehmen eines analytischen Verbrauchsmittels aus einem Vorratsbehältnis aufweist.

Besonders vorteilhaft ist die erfindungsgemäße Vorrichtung in solchen Analysegeräten einsetzbar, die netzunabhängig betrieben werden können, beispielsweise mittels Batterien oder Akkumulatoren. In solchen Geräten kann die Erfindung zur Lösung der Aufgabe, mit dem knappen Energievorrat der Batterien bzw. des Stromspeicherelements eine maximale Anzahl von analytischen Verbrauchsmitteln zu transportieren, besondere Vorteile bieten. Ferner können Anforderungen hinsichtlich der Begrenzung des Stromes, optimierte Fahrzeiten des Stößels oder eines geringen Platzbedarfs berücksichtigt und erfüllt werden, wobei die Herstellungskosten niedrig sind.

Die folgenden Ausführungsbeispiele der Erfindung lassen weitere vorteilhafte Merkmale und Besonderheiten erkennen, die anhand der Darstellung in den Zeichnungen im folgenden näher beschrieben und erläutert werden. Es zeigen:
- Fig. 1: einen Längsschnitt durch eine erfindungsgemäße Vorrichtung,
- Fig. 2: einen Querschnitt durch die Vorrichtung der Fig. 1,
- Fig. 3: einen Einzelheit zu Fig. 1,
- Fig. 4: eine Abwandlung zu Fig. 3,
- Fig. 5: die Vorrichtung der Fig. 1 in einer Grundstellung,
- Fig. 6: die Vorrichtung der Fig. 1 beim Durchstoßen einer Folie,
- Fig. 7: die Vorrichtung der Fig. 1 beim Verfahren eines Testelements in eine Meßposition,
- Fig. 8: die Vorrichtung der Fig. 1 beim Auswerfen des Testelements,
- Fig. 9: eine Wegdiagramm zu den Figuren 5 bis 8 und
- Fig. 10: ein Blockschaltbild einer erfindungsgemäßen Vorrichtung mit elektronischer Regelung.

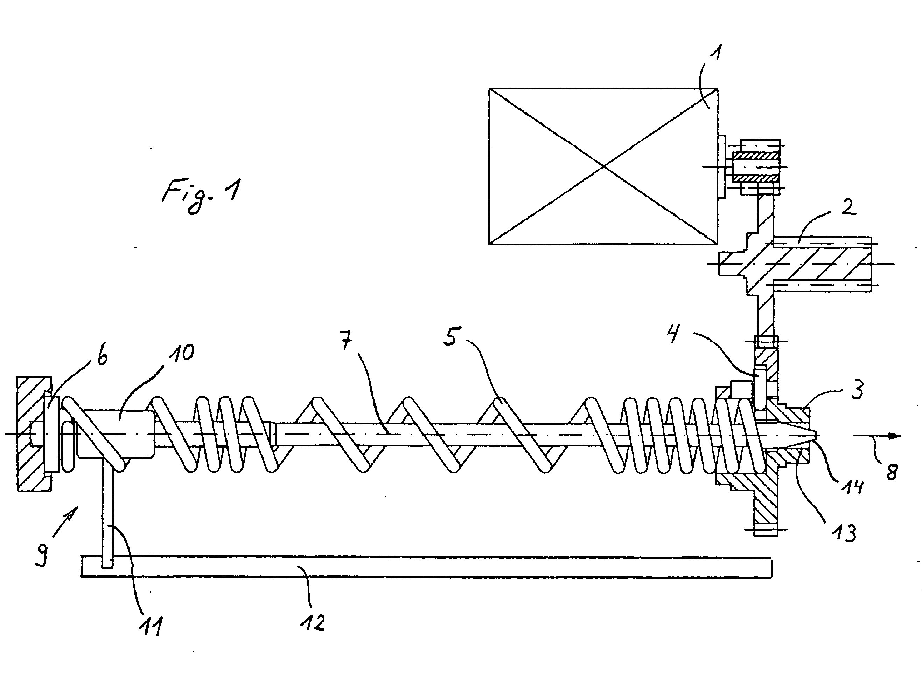

Die Fig. 1 zeigt eine bevorzugte Ausführungsform einer erfindungsgemäßen Vorrichtung zum Entnehmen eines analytischen Verbrauchsmittels aus einem in Fig. 1 nicht dargestellten Vorratsbehältnis, die die erfindungsgemäße Idee einer positionsabhängigen Vorschubkraft mittels einer mechanischen Kurvensteuerung realisiert und vorteilhaft für den Einsatz in sehr kleinen, batteriebetriebenen Analysegeräten geeignet ist. Die Vorrichtung umfaßt einen elektrischen Antriebsmotor 1, dessen Antriebskraft über ein Getriebe 2 auf ein Antriebsrad 3 übertragen wird. Mit dem Antriebsrad 3 ist der Mitnehmerzapfen 4 einer zylindrischen, schraubenförmig gewundenen Transportwendel 5 drehfest verbunden. Die Transportwendel 5 ist an ihrem anderen Ende in einem Drehlager 6 drehbar gelagert und kann durch den Antrieb in Drehung um ihre Längsachse versetzt werden.

Die Transportwendel 5 dient als Steuerelement einer mechanischen Weg- bzw. Kurvensteuerung und weist Abschnitte unterschiedlicher Steigung auf. Sie besteht beispielsweise aus Federstahl, da sie aus diesem Material vorteilhaft herstellbar ist. Es ist jedoch nicht erforderlich, daß die Transportwendel 5 zwischen dem Antriebsrad 3 und dem Drehlager 6 unter einer Zug- oder Druckspannung steht oder daß sie bei ihrer Funktion eine elastische Federkraft ausübt. Eine gewisse elastische Nachgiebigkeit kann zwar in manchen Anwendungsfällen zweckmäßig sein, ist jedoch nicht zwingend erforderlich. In den meisten Anwendungsfällen wird es zweckmäßig sein, die Transportwendel 5 aus einem relativ harten, wenig elastischen Federstahl möglichst starr, d.h. mit einer hohen Federkonstanten (Federrate) auszubilden.

Die Transportwendel 5 wird axial von einem Stößel 7 durchdrungen. In der Fig. 1 ist der Stößel 7 in der Ausgangsstellung dargestellt, in der er ganz in die Transportwendel 5 zurückgezogen ist. Zum Entnehmen eines analytischen Verbrauchsmittels aus einem Vorratsbehältnis wird der Stößel 7 aus der Transportwendel 5 in der durch den Pfeil 8 gekennzeichneten Richtung herausgeschoben. Um dabei die Drehbewegung der Transportwendel 5 in die Linearbewegung des Stößels 8 umzusetzen, weist der Stößel 7 ein Mitnehmerteil 9 auf, das eine dreh- und verschiebefest mit dem Stößel 7 verbundene Aufnahmebuchse 10 und einen an der Aufnahmebuchse 10 angeordneten Mitnehmerstift 11 umfaßt.

Die Außenabmessungen der Aufnahmebuchse 10 sind so klein, daß sie in axialer Richtung durch die Transportwendel 5 verschoben werden kann. Der Mitnehmerstift 11 ist vorzugsweise im wesentlichen quer zur Längsrichtung der Transportwendel 5, d.h. quer zur Vorschubrichtung des Stößels 7 angeordnet. Der Mitnehmerstift 11 ist in einer Mitnehmerführung 12 geführt, die sich parallel zu dem Stößel 7 erstreckt.

Wenn die Transportwendel 5 durch den Antrieb in Rotation versetzt wird, üben ihre Windungen eine Kraft auf den Mitnehmerstift 11 aus. Diese Kraft setzt sich aus einer Komponente, die den Mitnehmerstift 11 um den Stößel 7 dreht, und einer Komponente, die den Mitnehmerstift 11 in Richtung des Stößels 7 bewegt, zusammen. Die Drehbewegung des Mitnehmerstiftes 11 wird durch die Mitnehmerführung 12 unterbunden bzw. in speziellen Ausführungsformen geführt, so daß die verbleibende Kraftkomponente den Stößel 7 durch die Transportwendel 5 fördert.

Dabei ist es vorteilhaft, wenn die axialen Abmessungen der Aufnahmebuchse 10 so groß sind, daß sie immer sicher zwischen den Windungen geführt ist und sich nicht verklemmen kann. Die in axialer Richtung gemessene Länge der Aufnahmebuchse 10 sollte daher zweckmäßigerweise mindestens so groß wie der halbe maximale Abstand zweier Windungen der Transportwendel 5 sein. In diesem Fall genügt es, wenn der Stößel 7 in Vorwärtsrichtung in einer Buchse 13 geführt ist; die Führung am hinteren Ende wird dann durch die Transportwendel 5 und die darin axial verschiebbare Aufnahmebuchse 10 bewirkt. In anderen Ausführungsformen kann es aber auch zweckmäßig sein, wenn der Stößel 7 an einer weiteren Stelle, beispielsweise an seinem hinteren Ende gelagert ist. Hierzu kann beispielsweise der Stößel 7 einen axialen Hohlraum aufweisen, in den von seinem hinteren Ende ein Führungsdorn eingreift.

Zur Erhöhung der Lagestabilität der Transportwendel 5 und/oder des Stößels 7, d.h. zur Verhinderung oder Reduzierung einer seitlichen Auslenkung bzw. Verbiegung, kann auch ein anderes Führungselement vorgesehen sein. Hierzu kann beispielsweise eine die Transportwendel 5 innen oder außen abstützende, längsgeschlitzte Hülse dienen. Die Hülse kann gegebenenfalls auch die Funktion der Mitnehmerführung 12 erfüllen.

Durch eine entsprechende Ausbildung der Mitnehmerführung 12 kann die Rotationsposition des Stößels 7 bezüglich seiner Längsachse einfach und präzise definiert oder gesteuert werden. Dies ist beispielsweise dann besonders wichtig, wenn das vordere Ende des Stößels 7 in Form einer Klinge 14 ausgebildet ist, was vorteilhaft sein kann, um die Folie eines Vorratsbehältnisses ohne oder nur mit geringer Bildung von Schnipseln zu durchstoßen. Es kann dann wichtig sein, daß der Stößel 7 in einer bestimmten Orientierung zu dem aus dem Vorratsbehältnis zu entnehmenden analytischen Verbrauchsmittel steht, um einen sicheren Transport zu gewährleisten. Beispielsweise sollte bei streifenförmig ausgebildeten Testelementen die Klinge 14 etwa senkrecht zur Ebene der Teststreifen stehen, um eine sichere Förderung zu gewährleisten.

Die definierte Orientierung des Stößels 7 bzw. der Klinge 14 zu dem zu entnehmenden analytischen Verbrauchsmittel wird durch die Mitnehmerführung 12 bewirkt. Es ist sogar möglich, diese Relativpositionierung als Funktion des Transportweges zu variieren. Wenn die Mitnehmerführung 12 geradlinig ausgebildet ist, dreht sich der Stößel 7 während seines Vorschubs nicht. Wenn dagegen die Mitnehmerführung 12 in axialer Richtung gewunden ist, dreht sich der Stößel 7 in die jeweils durch die Mitnehmerführung 12 vorgegebene Lage.

Durch Umkehrung der Drehrichtung der Transportwendel 5 kehrt sich die Bewegungsrichtung des Stößels 7 ebenfalls um. Dabei kann je nach Ausführungsform des Mitnehmerteils 9 und der Steigung der Transportwendel 5 eine kurze Phase eintreten, in der sich die Transportwendel 5 dreht, ohne daß der Stößel 7 bewegt wird. Diese Phase endet, sobald wieder eine Windung der Transportwendel 5 an dem Mitnehmerstift 11 anliegt und dessen Verschiebung bewirkt. Die dadurch bedingte geringe Totzeit ist in der Praxis nicht störend und kann ggf. durch eine entsprechende Ausbildung der Kopplung zwischen Mitnehmerstift 11 und Transportwendel 5 reduziert oder verhindert werden. Ferner kann der Mitnehmerstift 11 am vorderen oder hinteren Ende der Transportwendel 5 angeordnete Endschalter betätigen, um am Ende des Transportweges den Transportvorgang zu beenden.

Die Transportwendel 5 weist über ihre Länge verschiedene Steigungen auf. Hierdurch ist es möglich, die von dem Stößel 7 ausgeübte Vorschubkraft sowie die Vorschubgeschwindigkeit des Stößels 7 in Abhängigkeit von seiner axialen Verschiebeposition zu variieren, auch wenn die Transportwendel 5 mit einer im wesentlichen konstanten Geschwindigkeit rotiert. In den Abschnitten mit einer geringen Steigung, in denen die Windungen der Transportwendel 5 eng beieinander liegen, ist die Vorschubkraft hoch und die Vorschubgeschwindigkeit gering. In den Abschnitten, in denen die Transportwendel 5 eine hohe Steigung aufweist und ihre Windungen einen großen Abstand haben, ist die Vorschubkraft gering und die Vorschubgeschwindigkeit hoch.

Folglich wird der Antriebsmotor 1 während der Vorschubbewegung des Stößels 7 viel gleichmäßiger belastet, als wenn die Transportwendel 5 eine gleichmäßige Steigung hätte. Dies hat zwei Vorteile. Einerseits wird die Batterie des Gerätes gleichmäßiger belastet, wodurch mehr Energie entnehmbar ist, da schädliche Stromspitzen vermieden werden. Andererseits kann der Antriebsmotor 1 auf einen optimalen Arbeitspunkt ausgelegt werden, so daß sich die Energieausnutzung verbessert. Insgesamt ergibt sich durch diese Maßnahme auch, daß die zur Bewegung des Stößels 7 für die Entnahme eines Verbrauchsmittels erforderliche Gesamtzeit reduziert ist.

Die variierende Steigung der Transportwendel 5 wirkt sich somit im Ergebnis wie ein wegabhängiges Getriebe aus, das auf den Stößel 7 während seiner Vorschubbewegung wirkende Laständerungen, die gerätespezifisch in Abhängigkeit von dem Vorschubweg vorgegeben sind, derart ausgeglichen werden, daß der Antriebsmotor 1 im wesentlichen gleichmäßig belastet wird.

Dies ist natürlich nur innerhalb bestimmter Toleranzen zu realisieren, wobei auch zu berücksichtigen ist, daß die im Einzelfall auftretenden Laständerungen sowie die Positionen, an denen sie auftreten, gewissen Schwankungen unterliegen. Auch wird es in praktischen Ausführungsformen hinnehmbar sein, wenn die Drehzahl des Antriebsmotors 1 im Laufe der Zeit, beispielsweise durch die Abnahme der verbleibenden Batteriekapazität, abnimmt, oder wenn der Antriebsmotor 1 bei der Vorschubbewegung des Stößels 7 geringen, verbleibenden Lastwechseln ausgesetzt ist. Die Höhe der Lastwechsel oder deren zeitliches Integral ist jedoch durch die erfindungsgemäße Ausbildung gegenüber konventionellen Vorrichtungen reduziert, so da ein erheblicher Vorteil in der praktischen Anwendung resultiert.

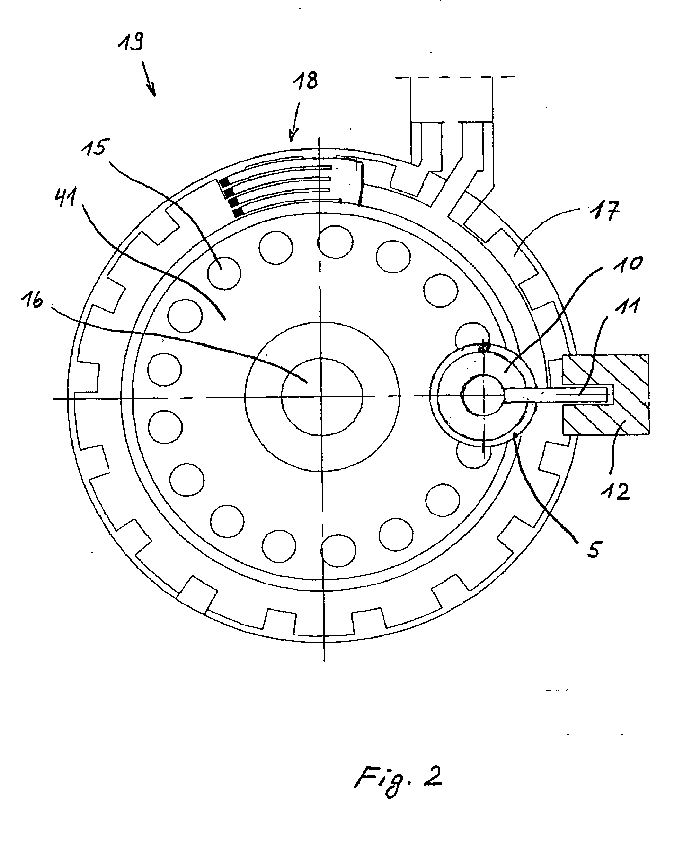

Die Fig. 2 zeigt einen Querschnitt durch eine erfindungsgemäße Vorrichtung. Dargestellt ist die Transportwendel 5, die Aufnahmebuchse 10 und der Mitnehmerstift 11 in einer geradlinig ausgebildeten Mitnehmerführung 12. Die Mitnehmerführung 12 ist im dargestellten Beispielsfall in Form einer Nut in einem Block realisiert. Andere Ausführungsformen, mit denen die Lage des Mitnehmerstiftes 11 bestimmt wird, sind durch den Fachmann in einfacher Weise zu realisieren.

In Fig. 2 ist ferner die Aufnahme- und Positioniervorrichtung 19 zum Aufnehmen eines Vorratsbehältnisses dargestellt, das siebzehn Kammern mit analytischen Verbrauchsmitteln aufweist. Entsprechend umfaßt die Aufnahmevorrichtung 19 siebzehn Durchstoßöffnungen 15 in einer Lochscheibe 41, die vor den Stößel 7 positioniert und durch die der Stößel 7 geführt werden kann. In der Mitte befindet sich ein Führungsstift 16, der in eine zentrale Bohrung des Vorratsbehältnisses eingreift.

Zum Drehen der Aufnahmevorrichtung 19 ist ein nicht dargestellter Antrieb vorgesehen, wobei die Positionierung mittels einer Positionierscheibe 17 und elektrischer Schleifkontakte 18 erfolgt. Das Vorratsbehältnis kann auf seine Außenseite einen Auswertecode aufweisen, der automatisch gelesen werden kann.

Der Führungsstift 16 greift in einem entsprechenden Meßgerät in die zentrale Bohrung des Vorratsbehältnisses ein und hält es in der richtigen Position für die Entnahme der Verbrauchsmittel. Am Rand der zentralen Bohrung kann sich beispielsweise ein Antriebszahnkranz an dem Vorratsbehältnis befinden, in den ein entsprechend geformtes Gegenstück beim Einsatz des Vorratsbehältnisses in ein Analysegerät eingreifen kann und mit dessen Hilfe das Vorratsbehältnis im Gerät rotiert wird. Durch die Rotation des Vorratsbehältnisses im Gerät kann es in entsprechend vordefinierte Positionen gebracht werden, so daß mit Hilfe des Stößels 7 die Entnahme und das Bereitstellen von Verbrauchsmitteln aus dem Vorratsbehältnis für Meßvorgänge ermöglicht wird.

Die Durchstoßöffnungen 15 sind kreisförmig, da auch der Schaft des Stößels 7 vorzugsweise einen kreisförmigen Querschnitt aufweist. Dies gilt jedoch nicht notwendigerweise auch für die Entnahmeöffnung oder Einschuböffnung in dem Vorratsbehältnis, die durch eine Folie verschlossen sind. Um ihre Fläche möglichst gering zu halten, sind diese Öffnungen häufig nicht kreisförmig, sondern weisen eine abweichende Form auf. Beispielsweise ist bei Teststreifen eine elliptische oder andere längliche Form vorteilhaft, wobei die Längserstreckung in Richtung der Teststreifenebene liegt.

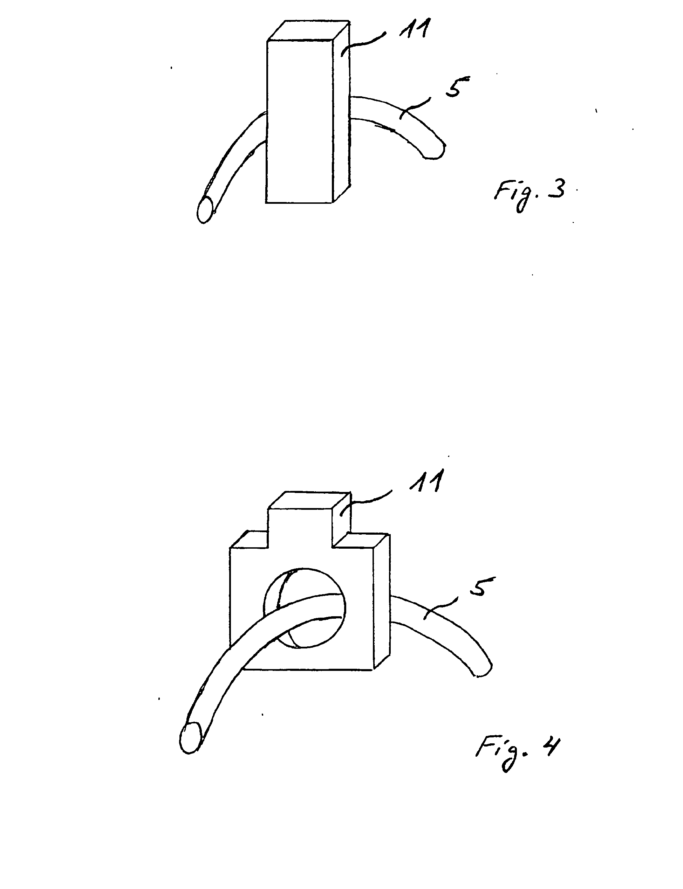

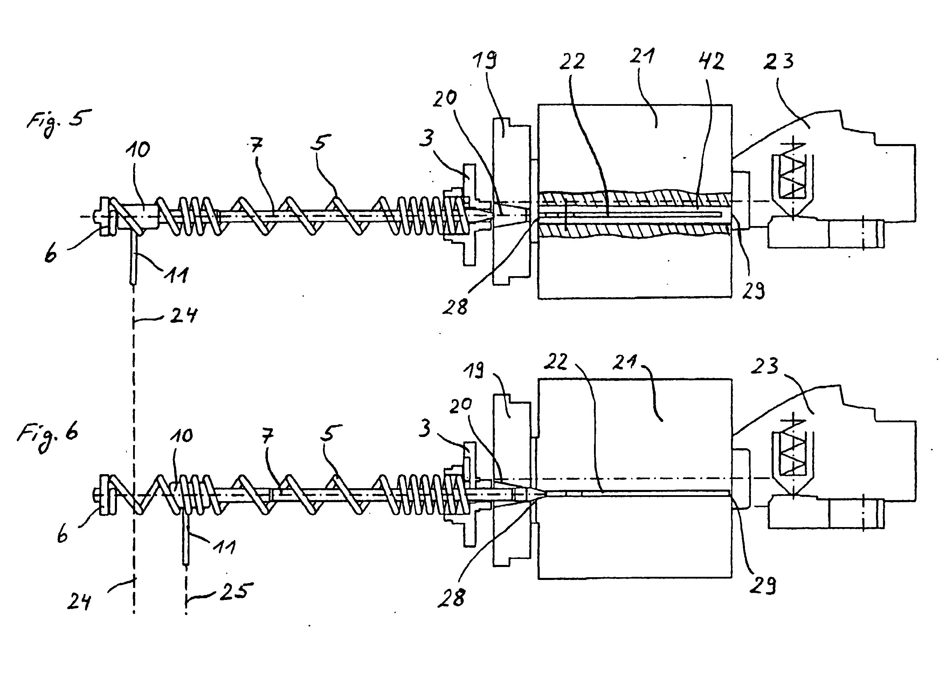

Die Figuren 3 und 4 veranschaulichen schematisch das Zusammenwirken des in der Mitnehmerführung 12 geführten Mitnehmerstiftes 11 und der Transportwendel 5. Die Fig. 5 entspricht dabei der Ausführung gemäß Fig. 1, in der die Transportwendel 5 an dem Mitnehmerstift 11 vorbeiläuft, so daß bei einer Umkehrung der Drehrichtung der Transportwendel 5 die Transportwendel 5 erst ein Stück gedreht werden muß, bevor der Stößel 7 zurückbewegt wird. In der Ausführungsform gemäß Fig. 4 ist die Transportwendel 5 durch eine Öffnung in dem Mitnehmerstift 11 geführt, wodurch der Totweg bei der Umkehrung der Drehbewegung verkleinert wird. In einer abgewandelten Ausführungsform kann beispielsweise auch vorgesehen sein, daß auf der Transportwendel 5 eine verschiebbare Laufbuchse angeordnet ist, die gelenkig an den Mitnehmerstift 11 angelenkt ist.

Die Fig. 5 bis 8 veranschaulichen verschiedene Phasen beim Betrieb der erfindungsgemäßen Vorrichtung gemäß Fig. 1. Dargestellt ist jeweils die Transportwendel 5 mit Stößel 7, die Aufnahmevorrichtung 19 mit Führungstrichter 20 sowie ein trommelförmiges Vorratsbehältnis 21, aus dem aus einer nur in der Fig. 5 dargestellten Kammer 42 ein Testelement 22 entnommen und in eine Meßvorrichtung 23 verfahren wird. Durch den Führungstrichter 20 kann der Stößel 7 in eine richtige Position relativ zu dem Vorratsbehältnis 21 gebracht werden.

In Fig. 5 befindet sich der Stößel 7 in einer Grundstellung 24, in der er in die Transportwendel 5 zurückgezogen ist. Ein Vorratsbehältnis 21 ist in die Aufnahmevorrichtung 19 eingesetzt und mittels einer Positioniereinrichtung wird die Kammer 42, die ein zu entnehmendes Testelement 22 enthält, vor den Stößel 7 gefahren. Die Einschuböffnung 28 und die gegenüberliegende Entnahmeöffnung 29 der Kammer 42, in der sich das Testelement 22 befindet, sind durch eine Folie verschlossen.

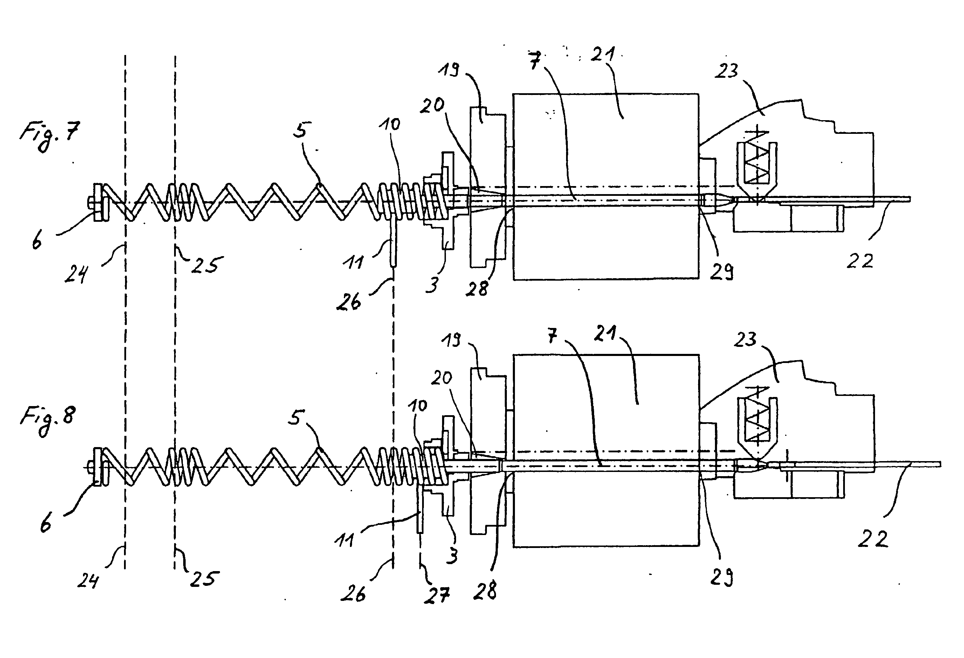

Die Transportwendel 5 wird dann mittels des Antriebs in Drehbewegung versetzt, wodurch sich der Stößel 7 vorwärts bewegt und beim in Fig. 6 dargestellten Foliendurchstoß 25 zunächst die Folie über der Einschuböffnung 28 durchstößt. Unmittelbar darauf wird die Folie an der Entnahmeöffnung 29 durch das Testelement 22 durchstoßen. Bei dem weiteren Transport des Testelements 22 in die in Fig. 7 dargestellte Gebrauchslage 26 wird der Stößel 7 weiter in das Vorratsbehältnis 21 hineingeschoben, wobei das Testelement 22 zunächst aus dem Vorratsbehältnis 21 herausgefördert und anschließend in einer definierten Position in der Meßvorrichtung 23 positioniert wird. In der Gebrauchslage 26 kann die Drehbewegung der Transportwendel 5 zur Durchführung der Messung unterbrochen werden.

In Fig. 8 ist dargestellt, wie nach Durchführung der Messung das Testelement 22 beim Auswurf 27 aus der Meßvorrichtung 23 durch einen weiteren Vorschub des Stößels 7 ausgestoßen wird. Anschließend wird der Stößel 7 in seine Grundstellung gemäß Fig. 5 zurückgefahren, wozu die Drehrichtung der Transportwendel 5 nach Auslösen eines Endschalters beim Auswurf 27 umgekehrt wird. Ein weiterer Endschalter stoppt dann die Rückzugsbewegung des Stößels 7 in der Grundstellung 24.

Beim Foliendurchstoß 25, beim Positionieren des Testelements 22 in der Gebrauchslage 26 und beim Auswurf 27 des Testelements muß der Stößel 7 eine erhöhte Vorschubkraft auf das Testelement 22 ausüben. Beim Anfahren des Stößels 7 aus der Grundstellung 24 zum Foliendurchstoß 25 sowie auf dem Weg zwischen Foliendurchstoß 25 und Positionieren in der Gebrauchslage 26 dagegen ist nur eine geringe Vorschubkraft erforderlich. Aus diesem Grund ist die Steigung der Transportwendel 5 in den verschiedenen Abschnitten unterschiedlich, so daß sich die von dem Stößel 7 bei seiner der Entnahme des Testelements 22 dienenden Vorschubbewegung ausgeübte Vorschubkraft in Abhängigkeit von der Position des Stößels 7 ändert. Wenn die Transportwendel 5 mit einer im wesentlichen konstanten Geschwindigkeit rotiert, ergibt sich zudem eine von der Position des Stößels 7 abhängige unterschiedliche Vorschubgeschwindigkeit.

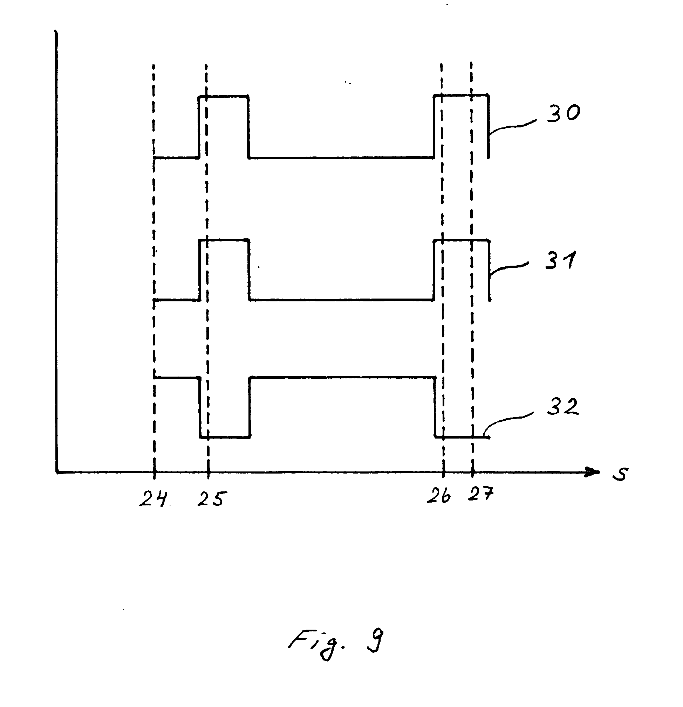

Dies ist in Fig. 9 veranschaulicht. In dem Diagramm sind die Vorschubkraft 30, das aufgebrachte Vorschubmoment 31 und die Vorschubgeschwindigkeit 32 des Stößels 7 in Abhängigkeit von seiner Position s für den in den Figuren 5 bis 8 dargestellten Ablauf veranschaulicht. Die Steigung der Transportwendel 7 ist proportional zur Vorschubgeschwindigkeit 32.

Zu erkennen ist, daß bereits kurz vor dem Foliendurchstoß 25 und der Gebrauchslage 26 die Vorschubkraft 30 erhöht und demzufolge die Vorschubgeschwindigkeit 32 reduziert wird und die Erhöhung bzw. Erniedrigung über eine gewisse Strecke aufrechterhalten wird, um den technischen Anforderungen beim Durchstoßen der Folie bzw. beim Positionieren in die Gebrauchslage 26 bzw. beim Auswurf 27 gerecht zu werden.

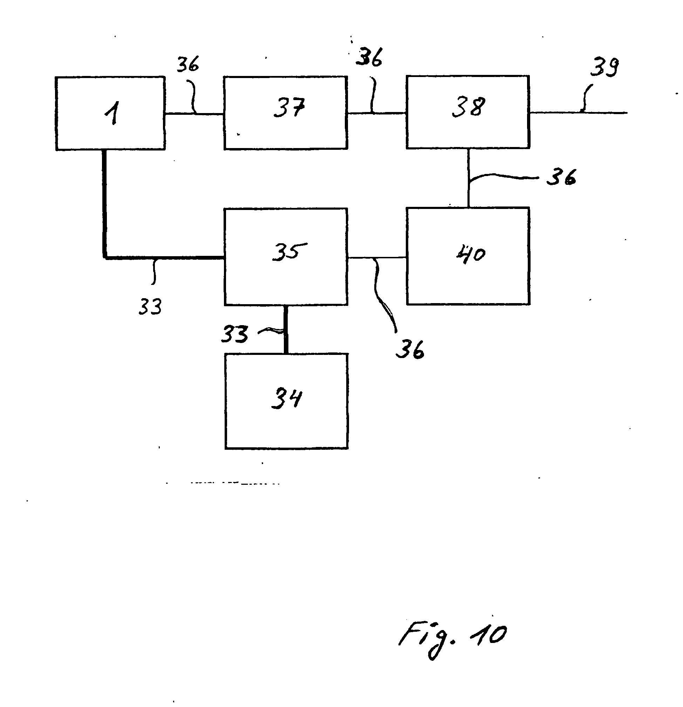

In dem Blockdiagramm der Fig. 10 ist veranschaulicht, wie die Vorschubkraft eines Stößels in Abhängigkeit von seiner Position auf rein elektronischem Wege realisiert werden kann. Hierzu wird der Energiefluß 33 von der Batterie 34 zu dem Antriebsmotor 1 gesteuert. Damit die Batterie bis zu einem hohen Grad entladen werden kann, wird ein Verstärker 35 eingesetzt, der bei einem niederohmigen Motor eine Strombegrenzung oder bei einem pulsweitenmodullierten Betrieb einen Ausgang mit Strombegrenzung aufweisen kann. Die Steuerung 36 erfolgt mittels eines Drehzahlmessers 37, eines Reglers 38 mit Sollgrößenvorgabe 39 und eines pulsweitenmodulierten oder linear geregelten Ausgangs 40. Diese Regelung ist jedoch nicht so effizient wie ein lastabhängiges, mechanisches Getriebe, da der Antriebsmotor 1 nicht stets in einem optimalen Arbeitspunkt gehalten werden kann.

### Bezugszeichenliste

- 1: Antriebsmotor
- 2: Getriebe
- 3: Antriebsrad
- 4: Mitnehmerzapfen
- 5: Transportwendel
- 6: Drehlager
- 7: Stößel
- 8: Pfeil
- 9: Mitnehmerteil
- 10: Aufnahmebuchse
- 11: Mitnehmerstift
- 12: Mitnehmerführung
- 13: Buchse
- 14: Klinge
- 15: Durchstoßöffnung
- 16: Führungsstift
- 17: Positionierscheibe
- 18: Schleifkontakt
- 19: Aufnahmevorrichtung
- 20: Führungstrichter
- 21: Vorratsbehälter
- 22: Testelement
- 23: Meßvorrichtung
- 24: Grundstellung
- 25: Foliendurchstoß
- 26: Gebrauchslage
- 27: Auswurf
- 28: Einschuböffnung
- 29: Entnahmeöffnung
- 30: Vorschubkraft
- 31: Vorschubmoment
- 32: Vorschubgeschwindigkeit
- 33: Energiefluß
- 34: Batterie
- 35: Verstärker
- 36: Steuerung
- 37: Drehzahlmesser
- 38: Regler
- 39: Sollgröße
- 40: Ausgang
- 41: Lochscheibe
- 42: Kammer
- s: Position

## Patentansprüche

1. Vorrichtung zum Entnehmen eines analytischen Verbrauchsmittels, insbesondere eines Testelements (22), aus einem Vorratsbehältnis (21),
wobei das Vorratsbehältnis (21) ein oder mehrere Kammern (42) aufweist, die Verbrauchsmittel enthalten, wobei die Kammern (42) jeweils eine Entnahmeöffnung (29) zum Entnehmen eines Verbrauchsmittels und eine der Entnahmeöffnung (29) gegenüberliegende Einschuböffnung (28) zum Einführen eines Stößels (7) für den Transport des Verbrauchsmittels aufweisen und die Entnahmeöffung (29) und die Einschuböffnung (28) zur Lagerung des Verbrauchsmittels mit einer Folie verschlossen sind,
die Vorrichtung umfassend
eine Antriebseinheit,
einen Stößel (7), der zur Entnahme eines Verbrauchsmittels mittels der Antriebseinheit verfahrbar ist, wobei die Größe der Vorschubkraft (30), die von dem Stößel (7) bei seiner der Entnahme eines Verbrauchsmittels dienenden Vorschubbewegung ausgeübt wird, in Abhängigkeit von der Position (s) des Stößels (7) steuerbar ist, und
eine mechanische Weg- bzw. Kurvensteuerung, die die Vorschubbewegung des Stößels (7) steuert,
**dadurch gekennzeichnet, daß**
die Weg- bzw. Kurvensteuerung ein schraubenförmig gewundenes, mittels der Antriebseinheit um seine Längsachse in Drehbewegung versetzbares und den Vorschub steuerndes Steuerelement umfaßt, das in Verbindung mit einem Mitnehmerteil steht, das den Stößel (7) beim Drehen des Steuerelementes in Vorschubrichtung bewegt.

2. Vorrichtung nach Anspruch 1**, dadurch gekennzeichnet, daß** die Vorschubkraft (30) in Abhängigkeit von dem Vorschubweg steuerbar ist, den der Stößel (7) zurückgelegt hat, und der Vorschubweg relativ zu einem Teil mit einer bezüglich der Vorrichtung festen Position bestimmbar ist.

3. Vorrichtung nach einem der vorhergehenden Ansprüche, **dadurch gekennzeichnet, daß** die Vorschubkraft (30) bei mindestens einem der folgenden Betriebszustände erhöht ist: Beim Durchstoßen (25) der Folie über der Einschuböffnung (28) durch den Stößel (7), beim Durchstoßen (25) der Folie über der Entnahmeöffnung (29) durch das Verbrauchsmittel, beim Positionieren des entnommenen Verbrauchsmittels in eine vorgegebene Gebrauchslage (26) oder beim Ausstoßen (27) eines verbrauchten Verbrauchsmittels aus einer vorgegebenen Gebrauchslage (26).

4. Vorrichtung nach einem der vorhergehenden Ansprüche, **dadurch gekennzeichnet, daß** die Vorschubgeschwindigkeit (32) des Stößels (7) in Bereichen mit erhöhter Vorschubkraft (30) reduziert und in Bereichen mit reduzierter Vorschubkraft (30) erhöht ist.

5. Vorrichtung nach einem der vorhergehenden Ansprüche, **dadurch gekennzeichnet, daß** die Vorschubbewegung des Stößels (7) mittels einer elektronischen Regelung der Antriebseinheit steuerbar ist.

6. Vorrichtung nach einem der vorhergehenden Ansprüche, **dadurch gekennzeichnet, daß** die Antriebseinheit einen Antriebsmotor (1) umfaßt, der mit einer im wesentlichen konstanten Antriebsleistung und/oder einer im wesentlichen konstanten Drehzahl betreibbar ist.

7. Vorrichtung nach einem der vorhergehenden Ansprüche, **dadurch gekennzeichnet, daß** sich die Längsachse des Steuerelements in Richtung der Vorschubbewegung des Stößels (7) erstreckt.

8. Vorrichtung nach Anspruch 7, **dadurch gekennzeichnet, daß** der Stößel (7) das Steuerelement axial durchdringt.

9. Vorrichtung nach einem der vorhergehenden Ansprüche, **dadurch gekennzeichnet, daß** die Steigung des Steuerelements längs seiner schraubenförmigen Windung konstant ist.

10. Vorrichtung nach einem der Ansprüche 1 bis 8, **dadurch gekennzeichnet**, die Steigung des Steuerelements längs seiner schraubenförmigen Windung entsprechend einer gewünschten Vorschubkraft -Positions -Abhängigkeit des Stößels (7) variabel ausgebildet ist.

11. Vorrichtung nach Anspruch 10, **dadurch gekennzeichnet, daß** die Drehzahl des Antriebsmotors (1) der Antriebseinheit und/oder die Drehzahl des Steuerelements beim Vorschub des Stößels (7) im wesentlichen konstant ist.

12. Vorrichtung nach einem der vorhergehenden Ansprüche, **dadurch gekennzeichnet, daß** das Steuerelement eine zylinderförmige Steuerwalze mit einer auf der Mantelfläche verlaufenden Nut und das Mitnehmerteil einen in die Nut eingreifenden Nutenstein umfaßt.

13. Vorrichtung nach einem der vorgehenden Ansprüche, **dadurch gekennzeichnet, daß** das Steuerelement eine zylindrische, schraubenförmig gewundene Transportwendel (5) umfaßt und das Mitnehmerteil als in die Windungen der Transportwendel (5) eingreifender Mitnehmerstift (11) ausgebildet ist.

14. Analysegerät zur Analyse einer medizinischen Probe mittels eines medizinischen Verbrauchsmittels, insbesondere zur Durchführung einer Analyse mittels eines Testelements (22), **dadurch gekennzeichnet, daß** es eine Entnahmevorrichtung nach einem der Ansprüche 1 bis 13 aufweist.

15. Analysehandgerät nach Anspruch 14, **dadurch gekennzeichnet, daß** es netzunabhängig betreibbar ist.

## Claims

1. Device for removing a consumable analytic product, in particular a test element, out of a storage container (21),
the storage container (21) having one or more chambers (42) containing the consumable product, wherein the chambers (42) each have a removal opening (29) for removal of a consumable product and an insertion opening (28) disposed opposite to the removal opening (29) for introduction of a plunger (7) to transport the consumable product, wherein the removal opening (29) and the insertion opening (28) are sealed with a foil for storing the consumable,
the device comprising
a drive unit,
a plunger (7), which is movable by the drive unit for removal of a consumable product, whereby the magnitude of the thrusting force (30) which is exercised by the plunger (7) during its forward motion for removal of a consumable product can be controlled in dependence on the position (s) of the plunger, and
a mechanical path control or a mechanical control having a cam or curved part which controls the thrusting motion of the plunger,
**characterized in that**
the path control comprises a helically wound control element which can be driven by the drive unit for rotation about its longitudinal axis to control the thrust and which is connected to a carrier member to move the plunger (7) in the thrusting direction during rotation of the control element.

2. Device according to claim 1, **characterized in that** the thrusting force (30) can be controlled in dependence on the thrusting path traveled through by the plunger (7), wherein the thrusting path can be determined relative to a component occupying a fixed location with respect to the device.

3. Device according to any one of the preceding claims, **characterized in that** the thrusting force (30) is increased during at least one of the following operational conditions: when the plunger (7) penetrates (25) through the foil covering the insertion opening (28), when the consumable product penetrates (25) through the foil covering the removal opening (29), when positioning the removed consumable product into a predetermined working position (26), or when expelling a used consumable product out of a predetermined working position (26).

4. Device according to any one of the preceding claims, **characterized in that** the thrusting speed (32) of the plunger (7) is reduced in regions of increased thrusting force (30) and increased in regions of reduced thrusting force (30).

5. Device according to any one of the preceding claims, **characterized in that** the thrusting motion of the plunger (7) can be controlled by electronic regulation of the drive unit.

6. Device according to any one of the preceding claims, **characterized in that** the drive unit includes a drive motor (1) which can be operated with substantially constant drive power and/or with a substantially constant rate of revolution.

7. Device according to any one of the preceding claims, **characterized in that** the longitudinal axis of the control element extends in the direction of thrusting motion of the plunger (7).

8. Device according to claim 7, wherein the plunger (7) passes through the control element in an axial direction.

9. Device according to any one of the preceding claims, **characterized in that** the pitch of the control element is constant along its helically shaped windings.

10. Device according to any one of claims 1 to 8, **characterized in that** wherein the pitch of the control element changes along its helically shaped winding in correspondence to a desired thrusting force-position dependence for the plunger (7).

11. Device according to claim 10, **characterized in that** the rate of revolution of the drive unit motor (1) and/or the rate of revolution of the control element is substantially constant during forward displacement of the plunger (7).

12. Device according to any one of the preceding claims, **characterized in that** the control element comprises a cylindrical drum controller having a groove in its outer surface and the carrier member comprises a tendon block engaging into the groove.

13. Device according to any one of the preceding claims, **characterized in that** the control element comprises a cylindrical helically wound transport spiral (5) and the carrier element is configured as a carrier pin (11) engaging into the windings of the transport spiral (5).

14. Analysis apparatus for analysis of a medical sample using a medical consumable product, in particular for carrying out an analysis using a test element, **characterized in that** it comprises a removal device according to any one of claims 1 to 13.

15. Analysis apparatus according to claim 14, **characterized in that** wherein it can be operated independent of the power mains.

## Revendications

1. Dispositif pour retirer un moyen consommable d'analyse, en particulier un élément de test (22), d'un récipient de stockage (21),
> le récipient de stockage (21) présentant une ou plusieurs chambres (42) qui contiennent des moyens consommables, les chambres (42) présentant chacune une ouverture de prélèvement (29) pour retirer un moyen consommable et une ouverture d'introduction (28) opposée à l'ouverture de prélèvement (29) pour introduire un coulisseau (7) pour le transport du moyen consommable et l'ouverture de prélèvement (29) et l'ouverture d'introduction (28) pour le stockage du moyen consommable étant fermées avec une feuille, lequel dispositif comprend
> une unité d'entraînement,
> un coulisseau (7), qui peut être déplacé pour retirer un consommable au moyen de l'unité d'entraînement, la grandeur de la force d'avancement (30), qui est exercée par le coulisseau (7) lors de son mouvement d'avancement servant au prélèvement d'un moyen consommable, pouvant être commandée en fonction de la position (s) du coulisseau (7), et
> une commande de course et de courbe mécanique, qui commande le mouvement d'avancement du coulis-seau (7),
**caractérisé en ce que**
> la commande de course ou de courbe comprend un élément de commande tourné en forme de vis, pouvant être mis en mouvement de rotation autour de son axe longitudinal au moyen de l'unité d'entraînement et commandant l'avancement, qui est en liaison avec une partie d'entraînement qui déplace le coulisseau (7) lors de la rotation de l'élément de commande dans le sens d'avancement.

2. Dispositif selon la revendication 1, **caractérisé en ce que** la force d'avancement (30) peut être commandée en fonction de la course d'avancement que le coulisseau (7) a parcourue et la course d'avancement peut être déterminée par rapport à une partie avec une position fixe par rapport au dispositif.

3. Dispositif selon l'une quelconque des revendications précédentes, **caractérisé en ce que** la force d'avancement (30) est augmentée lors d'au moins l'un des états de service suivants : lors de la traversée (25) de la feuille au-dessus de l'ouverture d'introduction (28) par le coulisseau (7), lors de la traversée (25) de la feuille au-dessus de l'ouverture de prélèvement (29) par le moyen consommable, lors du positionnement du moyen de consommation prélevé dans une position d'utilisation (26) prédéfinie ou lors de l'expulsion (27) d'un consommable consommé à partir d'une position d'utilisation (26) prédéfinie.

4. Dispositif selon l'une quelconque des revendications précédentes, **caractérisé en ce que** la vitesse d'avancement (32) du coulisseau (7) est réduite dans des zones présentant une force d'avancement (30) élevée et augmentée dans des zones présentant une force d'avancement (30) réduite.

5. Dispositif selon l'une quelconque des revendications précédentes, **caractérisé en ce que** la vitesse d'avancement du coulisseau (7) peut être contrôlée au moyen d'un réglage électronique de l'unité d'entraînement.

6. Dispositif selon l'une quelconque des revendications précédentes, **caractérisé en ce que** l'unité d'entraînement comprend un moteur d'entraînement (1) qui peut être exploité avec une puissance d'entraînement sensiblement constante et/ou un régime sensiblement constant.

7. Dispositif selon l'une quelconque des revendications précédentes, **caractérisé en ce que** l'axe longitudinal de commande s'étend en direction du mouvement d'avancement du coulisseau (7).

8. Dispositif selon la revendication 7, **caractérisé en ce que** le coulisseau (7) traverse axialement l'élément de commande.

9. Dispositif selon l'une quelconque des revendications précédentes, **caractérisé en ce que** la pente de l'élément de commande est constante le long de sa spire hélicoïdale.

10. Dispositif selon l'une quelconque des revendications 1 à 8, **caractérisé en ce que** la pente de l'élément de commande le long de sa spire hélicoïdale est conçue variable en fonction d'une dépendance force d'avancement-position souhaitée du coulisseau (7).

11. Dispositif selon la revendication 10, **caractérisé en ce que** le régime du moteur d'entraînement (1) de l'unité d'entraînement et/ou le régime de l'élément de commande est sensiblement constant lors de l'avancement du coulisseau (7).

12. Dispositif selon l'une quelconque des revendications précédentes, **caractérisé en ce que** l'élément de commande comprend un cylindre de commande de forme cylindrique avec une rainure agencée sur la surface d'enveloppe et la partie d'entraînement comprend un coulisseau s'engageant dans la rainure.

13. Dispositif selon l'une quelconque des revendications précédentes, **caractérisé en ce que** l'élément de commande comprend une hélice de transport (5) cylindrique et tourné en forme d'hélice et la partie d'entraînement est conçue comme une broche d'entraînement (11) s'engageant dans les spires de l'hélice de transport (5).

14. Appareil d'analyse pour l'analyse d'un échantillon médical au moyen d'un produit consommable médical, en particulier pour la mise en oeuvre d'une analyse au moyen d'un élément de test (22), **caractérisé en ce qu'**il présente un dispositif de prélèvement selon l'une quelconque des revendications 1 à 13.

15. Appareil d'analyse selon la revendication 14, **caractérisé en ce qu'**il peut être exploité de façon indépendante du réseau.
